# EUROPEAN PATENT SPECIFICATION

(11) **EP 0 724 558 B1**
(45) Date of publication and mention of the grant of the patent: **29.04.1998**
(21) Application number: 94926212.5
(22) Date of filing: 18.08.1994
(51) Int. Cl.: C07C 67/38, C07C 69/54

(54) **PROCESS FOR THE CARBONYLATION OF ACETYLENICALLY UNSATURATED COMPOUNDS**
VERFAHREN ZUR CARBONYLIERUNG VON ACETYLENISCH UNGESÄTTIGTEN VERBINDUNGEN
PROCEDE DE CARBONYLATION DE COMPOSES ACETYLENIQUEMENT INSATURES

(30) Priority: 19.08.1993 EP 93202446
(43) Date of publication of application: 07.08.1996
(73) Proprietor: SHELL INTERNATIONALE RESEARCH MAATSCHAPPIJ B.V., 2596 HR Den Haag (NL)
(72) Inventor: DRENT, Eit, NL-1031 CM Amsterdam (NL); JAGER, Willem, Wabe, NL-1031 CM Amsterdam (NL); SUYKERBUYK, Jacoba, Catherina, Lucia, Johanna, NL-1031 CM Amsterdam (NL)
(86) International application number: EP9402763
(87) International publication number: WO9505357

(56) References cited:
- EP-A- 0 271 144
- EP-A- 0 386 833
- EP-A- 0 392 601
- "Carbonylation: Direct synthesis of Carbonyl Compounds" HM Colquhoun, DJ Tompson and MV Twigg (1991) pgs 45-47, 106, 107, 227-246. "New Synthesis with Carbon Monoxide" J. Falbe (1980) pgs 250-256 & 263-268.

## Description

The invention relates to a process for the carbonylation of acetylenically unsaturated compounds, whereby a feedstock, comprising an acetylenically unsaturated compound and a relatively minor amount of an 1,2-alkadiene compound, is contacted under carbonylation conditions with carbon monoxide and a hydroxylated co-reactant.

A problem, encountered with the processes for the carbonylation of acetylenically unsaturated compounds, consists in that the available feedstocks for these processes generally contain, in addition to the acetylenically unsaturated compound, 1,2-alkadiene compounds (so-called allenes). The presence of 1,2 alkadiene compounds, even in relatively small amounts, unfavourably affects the catalyst activity. Small quantities of 1,2-alkadiene impurities (up to 0.4%) can often be tolerated. However, the amounts commonly present in the acetylenic feedstocks require that special measures are taken, before they can be used for the carbonylation process.

For instance, in EP-A-0 271 144 a process is described for the carbonylation of acetylenically unsaturated compounds in the presence of highly active catalyst systems, without commenting on or pointing toward catalyst systems that overcome the problem of catalyst poisoning due to the presence of 1,2-alkadiene in the feedstock.

A related carbonylation process is disclosed in EP-A-0 386 833. According to this document, alpha-unsaturated hydrocarbons, in particular propyne, can be converted with remarkably high selectivity into beta-carbonylated products by using a catalyst system comprising a Group VIII metal, and certain phosphines. It is stated that for a high reaction rate, the phosphine component of the catalyst should contain a 2-pyridyl group that is substituted with preferably a hydrocarbyl group at the 6-position. Although carbonylation of feedstock containing up to 0.4% of 1,2-alkadiene is exemplified, no direction is given as regards the phosphine component when higher amounts of 1,2-alkadiene are present.

In EP-A-0,441,446 a carbonylation catalyst system is disclosed that performs adequately under basic conditions. The catalyst system comprises: a) a source of a Group VIII metal (e.g., a palladium compound); b) a (mono- or bidentate) (di)phosphine having an aromatic substituent which contains an imino nitrogen atom, e.g., an optionally substituted 2-pyridyl group; c) a source of protons; and d) a tertiary amine. The illustrated basic catalyst systems (based on phosphines bearing 2-pyridyl and 6-methyl-2-pyridyl groups) exhibit improved tolerance towards allenes (up to 7%, example 18), provided a tertiary amine is present (see comparative example G versus examples 12 and 15). However, no information may be gained from this document that further improvements in respect of the carbonylation process, even carried out in the absence of the tertiary amine, could be achieved.

Surprisingly it has now been found that by employing certain specific mono- or bidentate (di)phosphine ligands, the tolerance with respect to allenes is further improved.

This selection moreover allows the carbonylation to be performed under non-basic conditions, starting from conventional feedstocks that are contaminated with 1,2-alkadiene. Specific measures for the removal of 1,2-alkadiene compounds such as disclosed in EP-A-0 392 601 that precede the carbonylation reaction, are thereby made redundant.

The invention may be defined as relating to a process for the carbonylation of acetylenically unsaturated compounds, whereby a feedstock comprising an acetylenically unsaturated compound and an amount of an 1,2-alkadiene compound in a molar ratio of 0.004 to 0.1 is contacted under carbonylation conditions with carbon monoxide and a hydroxylated co-reactant, in the presence of a catalyst system based on:
a) a source of cations of one or more metals of Group VIII of the Periodic Table;
b) a phosphine of the general formula PR¹R²R³ or R²R³M-R-PR¹R², wherein R¹ represents a heteroaryl group of the formula 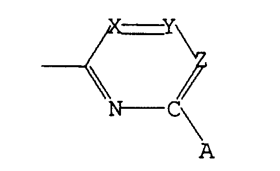 wherein A represents a functional group that is electron-withdrawing relative to hydrogen, and each of X, Y and Z independently represents a nitrogen atom or a C-Q group, whereby Q is a hydrogen atom or a substituent, R² and R³ independently represent a substituted or non-substituted (hetero)hydrocarbyl group or have the aforesaid meaning of R¹, M is an element of Group VIa, preferably a nitrogen or phosphorus atom, R represents a bridging (substituted) hydrocarbyl group having 1 to 4 carbon atoms in the bridge; and
c) a protonic acid.

The catalyst system used in the process of the invention is based, as regards a), on a source of cations of one or more metals of Group VIII. These metals include iron, cobalt, nickel, ruthenium, rhodium, palladium, iridium, osmium, and platinum. Preferably, the catalyst system is based on a source of palladium cations.

The source of cations of metals of Group VIII may be the metallic element or a metal compound, such as a metal salt or a complex of the metal with a phosphine, with carbon monoxide or with acetylacetonate. It is advantageously a metal compound, in particular a metal salt. Examples of suitable metal salts are salts of sulphuric acid, nitric acid, sulphonic acids, phosphonic acids, perhalic acids and carboxylic acids, such as alkane carboxylic acids with 1 to 12 carbon atoms, for example acetic acid and propionic acid, or halogenated carboxylic acids, for example trichloroacetic acid and trifluoroacetic acid. Palladium acetate has proved to be a particularly suitable source of metal cations.

In the phosphines of the catalyst system (component b), each of R² and R³ preferably represents a substituted or unsubstituted pyridyl, alkyl or aryl group. Examples of suitable R² and R³ groups are 2-pyridyl, phenyl, tolyl-, xylyl, and cyclohexyl groups and alkyl groups having from 3 to 7 carbon atoms. Phosphines wherein both R² and R³ represent a phenyl group are preferred.

The functional group(s) A may for instance be a trialkylammonium, a nitro, cyano, sulphonic, carbonic, carbonyl, or a haloalkyl group or a halogen atom (e.g., any of the groups listed in "Organic Chemistry", by Morrison and Boyd, 3rd ed. 1980, p. 360.). Other electron-withdrawing groups will be known to the skilled man. Preferably (for ease of manufacturing the ligand) it is selected from the group consisting of halogen atoms and haloalkyl groups with one to three carbon atoms. More preferably, A represents a halogen atom such as a bromine, fluorine or chlorine atom. Phosphines, in which (each of) the functional group(s) A is a chlorine atom, are most preferred.

Examples of phosphines, wherein X, Y and/or Z represents a nitrogen atom are phosphines substituted with a pyrimidinyl-, pyrazinyl-, s-triazinyl- and as-triazinyl group.

Preferably, the phosphine is substituted with a 2-pyridyl group, i.e. X, Y and Z each represents a group C-Q, in which Q is a hydrogen atom or a substituent.

Suitable substituents represented by Q include electron-withdrawing substituents, in particular the substituents as indicated above with respect to A. Advantageously, Q represents electron-donating substituents, such as alkyl groups, for example methyl and ethyl groups, amino and (di)alkylamino groups.

Preferably, the phosphine ligand is a monophosphine of the general formula PR¹R²R³.

The function of the protonic acid on which the catalyst system further is based (component c), is believed to provide a source of protons. The protonic acid may be generated in situ.

Preferably the protonic acid has a substantially non-coordinating anion, i.e. an anion which does not, or only to a very minor extent, coordinate with the metal of Group VIII. Preferred acids in this respect are sulphuric acid, sulphonic acids and halogenated carboxylic acids.

Examples of suitable protonic acids are optionally substituted alkylsulphonic acids, such as methanesulphonic acid, trifluoromethanesulphonic acid, tert-butylsulphonic acid, perhalic acids, such as perchloric acid, and acidic ion exchange resins, such as a sulphonated ion exchange resin.

The number of moles of phosphine and of moles of protonic acid per mole atoms of metal of Group VIII may vary considerably. Recommended phosphine amounts are in the range of 10 to 100 moles of phosphine per mole atom of metal of Group VIII and in particular in the range of 20 to 80. The amount of protonic acid is preferably selected such that per mole atom of metal of Group VIII, 2 to 500 moles of protonic acid are present.

The catalyst system of the invention may be homogeneous or heterogeneous, but is preferably homogeneous. The amount in which the catalyst is applied is suitably selected such that per mole of acetylenically unsaturated compound to be converted, from 10⁻⁸ to 10⁻¹ mole atoms of Group VIII metal is present, preferably from 10⁻⁷ to 10⁻² on the same basis.

Suitable acetylenically unsaturated compounds, to be used as starting material in the process of the invention, include optionally substituted alkynes with 2 to 20 carbon atoms per molecule. Examples are acetylene, propyne, 1-butyne, 2-butyne, 1-hexyne, phenyl acetylene and benzylethyne. Preferably, unsubstituted alkynes with 3 to 10 carbon atoms are used.

In view of the industrial outlets for the carbonylated products, propyne is a preferred starting material.

As has been stated above, a major advantage of the catalyst systems of the invention consists in their tolerance towards 1,2-alkadiene compounds in the acetylenic feedstocks. Accordingly, commercially available feedstocks may be used that containing small amounts of 1,2-alkadiene compounds, such as propadiene, in addition to the acetylenically unsaturated compounds. In general, a 1,2-alkadiene content of at most 10%, based on acetylenically unsaturated compound, can be tolerated. It is recommended to use feedstocks in which the amount of 1,2-alkadiene compounds is lower, suitably in the range of 0.002 to 0.05 moles per mole of acetylenically unsaturated compound.

The hydroxylated co-reactant may be any hydroxyl-containing compound such as a monohydric, dihydric or polyhydric alkanol, a phenol, or water.

Monohydric alkanols are preferred, in particular those having from 1 to 4 carbon atoms. Among these, methanol is most preferred.

The co-reactant is suitably used in excess, thereby avoiding the need of a separate diluent or solvent. However, a liquid diluent may be applied, if so desired. Preferably, non-alkaline diluents are used, such as ketones, e.g. methylisobutylketone, or ethers, e.g. dipropylether or 2,5,8-trioxanonane (diglyme).

Owing to the high activity of the catalysts, the process of the invention proceeds readily at moderate reaction conditions. Suitable reaction temperatures are, for instance, in the range of 20 to 100 °C, preferably in the range of 30 to 75 °C.

The reaction pressure is usually selected in the range of 1 to 100 bar. Preferably, the pressure is in the range of 5 to 70 bar.

The invention is illustrated with the following, non-limiting examples.

### Examples

All experiments were carried out in a 250 ml "Hastelloy C" (trade mark) magnetically stirred autoclave. The autoclave was charged with 0.025 mmoles of palladium(II) acetate, the selected diphenyl(2-pyridyl)phosphine and protonic acid in the amounts indicated hereafter, and 50 ml of methanol.

Air was evacuated from the autoclave, whereupon 30 ml of a propadiene-containing propyne feed was added.

Subsequently, carbon monoxide was supplied up to a pressure of 60 bar. The autoclave was sealed and heated to the desired reaction temperature.

As soon as the falling pressure remained constant (marking the completion of the reaction), the contents of the autoclave were cooled and a sample was withdrawn and analysed by gas liquid chromatography.

### Example I

a) An experiment was carried out in the manner as outlined above, whereby as phosphine 1 mmol of biphenyl (6-chloro-2-pyridyl)phosphine and as protonic acid 2 mmol of trifluoromethanesulphonic acid was used. The feed was propyne, containing 0.2% of propadiene. The reaction temperature was 45 °C.
   The reaction time (completion) was 0.16 hour. Analysis showed that methyl methacrylate had been formed with a selectivity of 99.88% at a propyne conversion of about 100%. The average reaction rate was calculated to be 73,000 moles of product per mole atom of palladium and per hour.
b) The experiment described under a) was repeated with the difference that the propyne feed contained 2.2% of propadiene.
   The reaction time was 0.5 hour. The average reaction rate was calculated to be 24,000 moles of product per mole atom of palladium and per hour.

### Example II

An experiment was carried out substantially as described in Example I with the following differences:
i) the propyne feed contained 4.5% of propadiene;
ii) 2 mmol of biphenyl(6-chloro-2-pyridyl)phosphine were used; and
iii) the reaction temperature was 50 °C.

The reaction time was 2.5 hours. The average reaction rate was calculated to be 5,500 moles of product per mole atom of palladium and per hour.

### Example III

An experiment was carried out substantially as described in Example II, with the following differences:
i) the propyne feed contained 4.7% of propadiene;
ii) 3 mmol of biphenyl(6-chloro-2-pyridyl)phosphine were used; and
iii) as protonic acid 2 mmol of tert-butylsulphonic acid was used.

The reaction time was 1 hour. The average reaction rate was calculated to be 8,900 moles of product per mole atom of palladium and per hour.

### Example IV

An experiment was carried out substantially as described in Example III, with the following differences:
i) the propyne feed contained 7.0% of propadiene;
ii) as protonic acid 3 mmol of methanesulphonic acid was used; and
iii) the reaction temperature was 60 °C.

The reaction time was 0.5 hour. The average reaction rate was calculated to be 20,000 moles of product per mole atom of palladium and per hour. Analysis showed that the propyne conversion was >95% and that the reaction mixture contained 5% of propadiene, calculated on residual propyne, indicating that most of the propadiene had been converted into methyl methacrylate product.

### Example V

a) An experiment was carried out, substantially as described in Example I(a), with the following differences:
   i) 1 mmol of biphenyl(6-bromo-2-pyridyl)phosphine was used;
   ii) as protonic acid 2 mmol of methanesulphonic acid was used; and
   iii) the reaction temperature was 30 °C.
      The reaction time was 1.5 hour. The average reaction rate was calculated to be 9,200 moles of product per mole atom of palladium and per hour.
b) The experiment described under a) was repeated with the following differences:
   i) the propyne feed contained 1.7% of propadiene;
   ii) as protonic acid 2 mmol of trifluoromethanesulphonic acid was used; and
   iii) the reaction temperature was 45 °C.
      The reaction time was 2.5 hours. The average reaction rate was calculated to be 6,000 moles of product per mole atom of palladium and per hour.

### Example IV

An experiment was carried out, substantially as described in Example I(a), with the following differences:
i) 1 mmol of biphenyl(6-trifluoromethyl-2-pyridyl)phosphine and 1 mmol of biphenyl(2-pyridyl)phosphine were used;
ii) as protonic acid 3 mmol of trifluoromethanesulphonic acid was used; and
iii) the reaction temperature was 35 °C (with an exotherm of 60 °C).

The reaction time was 1 hour. Analysis showed that methyl methacrylate had been formed with a selectivity of 98.6%. The average reaction rate was calculated to be 100,000 moles of product per mole atom of palladium and per hour.

### Example A (for comparison, not according to the invention)

a) An experiment was carried out, substantially as described in Example V(a), with the following differences:
   i) 1 mmol of biphenyl(2-pyridyl)phosphine was used; and
   ii) the reaction temperature was 30 °C.
      The reaction time was 2 hours. The average reaction rate was calculated to be 2,500 moles of product per mole atom of palladium and per hour.
b) The experiment described under a) was repeated with the following differences:
   i) the propyne feed contained 2.3% of propadiene; and
   ii) the reaction temperature was 50 °C.

The reaction time was 5 hours. The average reaction rate was calculated to be 625 moles of product per mole atom of palladium and per hour.

### Example B (for comparison, not according to the invention)

a) An experiment was carried out, substantially as described in Example V(a), with the following differences:
   i) 1 mmol of biphenyl (3-methyl-2-pyridyl)phosphine was used; and
   ii) the reaction temperature was 50 °C.
      The reaction time was 5 hours. The average reaction rate was calculated to be 7,100 moles of product per mole atom of palladium and per hour.
b) The experiment described under a) was repeated with the difference that the propyne feed contained 2.3% of propadiene.

The average reaction rate was calculated to be 800 moles of product per mole atom of palladium and per hour.

### Example C (for comparison, not according to the invention)

a) An experiment was carried out, substantially as described in Example V(a), with the following differences:
   i) 1 mmol of biphenyl (3-chloro-2-pyridyl)phosphine was used; and
   ii) the reaction temperature was 35 °C.
      The reaction time was 0.25 hour. The average reaction rate was calculated to be 50,000 moles of product per mole atom of palladium and per hour.
b) The experiment described under a) was repeated with the following differences:
   i) the propyne feed contained 2.3% of propadiene; and
   ii) the reaction temperature was 50 °C.

The reaction time was 5 hours. The average reaction rate was calculated to be 2,100 moles of product per mole atom of palladium and per hour.

### Example D (for comparison, not according to the invention)

a) An experiment was carried out, substantially as described in Example V(a), with the following differences:
   i) 1 mmol of biphenyl (5-chloro-2-pyridyl)phosphine was used; and
   ii) the reaction temperature was 35 °C.
      The reaction time was 0.1 hour. The average reaction rate was calculated to be 175,000 moles of product per mole atom of palladium and per hour.
b) The experiment described under a) was repeated with the following differences:
   i) the propyne feed contained 2.3% of propadiene; and
   ii) the reaction temperature was 50 °C.

The reaction time was 5 hours. The average reaction rate was calculated to be 3,300 moles of product per mole atom of palladium and per hour.

### Example E (for comparison, not according to the invention)

An experiment was carried out, substantially as described in Example A(b), with the difference that as phosphine biphenyl(6-methyl-2-pyridyl)phosphine was used.

The average reaction rate was calculated to be 400 moles of product per mole atom of palladium and per hour.

### Conclusions

As can be seen from Example A, with a non-substituted phosphine as catalyst component, a reaction rate of only 2,500 moles per mole atom Pd per hour is obtained, even when a feedstock having a relatively low (0.2%) propadiene content is used. Contrarily, the presence of substituted phosphines in the catalyst systems, used in Examples B, C, and D, results in considerably higher reaction rates in converting feedstocks having a low propadiene content.

However, as shown in Examples B(b), C(b) and D(b), the activity of these catalysts considerably decreases by the presence of propadiene in the feedstock.

In contrast, the use of the catalysts described in Examples I to VI, for the conversion of feedstocks containing 2% or more of propadiene, still results in high reaction rates.

## Claims

1. A process for the carbonylation of acetylenically unsaturated compounds, whereby a feedstock comprising an acetylenically unsaturated compound and an amount of a 1,2-alkadiene compound in a molar ratio of 0.004 to 0.1 is contacted under carbonylation conditions with carbon monoxide and a hydroxylated co-reactant, in the presence of a catalyst system based on:
a) a source of cations of one or more metals of Group VIII of the Periodic Table;
b) a phosphine of the general formula PR¹R²R³ or R²R³M-R-PR¹R², wherein R¹ represents a heteroaryl group of the formula 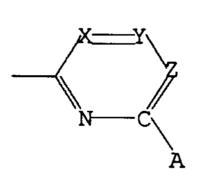 wherein A represents a functional group that is electron-withdrawing relative to hydrogen, and each of X, Y and Z independently represents a nitrogen atom or a C-Q group, whereby Q is a hydrogen atom or a substituent, R² and R³ independently represent a substituted or non-substituted (hetero)hydrocarbyl group or have the aforesaid meaning of R¹, M is an element of Group VIa, preferably a nitrogen or phosphorus atom, R represents a bridging (substituted) hydrocarbyl group having 1 to 4 carbon atoms in the bridge; and
c) a protonic acid.

2. A process as claimed in claim 1, wherein the catalyst system is based on a phosphine wherein R² and R³ independently represent a substituted or non-substituted pyridyl, alkyl or aryl group, preferably a phenyl group.

3. A process as claimed in claim 1 or 2, wherein the catalyst system is based on a phosphine wherein A represents a substituent selected from the group consisting of halogen atoms and haloalkyl groups with one to three carbon atoms.

4. A process as claimed in claim 3, wherein A represents a halogen atom, preferably a chlorine atom.

5. A process as claimed in any of claims 1 to 4, wherein the catalyst system is based on a phosphine wherein R¹ represents a 6-A-2-pyridyl group.

6. A process as claimed in any of claims 1 to 5, wherein the catalyst system is based on a monophosphine, preferably on biphenyl (6-chloro-2-pyridyl)phosphine.

7. A process as claimed in any one of claims 1 to 6, carried out in the absence of a tertiary amine.

8. A process as claimed in any of claims 1 to 7, wherein the molar amount of 1,2-alkadiene compound in the feedstock per mole of acetylenically unsaturated compound is in the range of 0.004 to 0.05.

9. A process as claimed in any of claims 1 to 8, wherein methyl methacrylate is prepared by reacting a feedstock comprising propyne and 1,2-propadiene, with carbon monoxide and methanol.

## Patentansprüche

1. Verfahren zur Carbonylierung von acetylenisch ungesättigten Verbindungen, worin ein Einsatzmaterial, das eine acetylenisch ungesättigte Verbindung und eine Menge einer 1,2-Alkadienverbindung in einem Molverhältnis von 0,004 bis 0,1 umfaßt, unter Carbonylierungsbedingungen mit Kohlenmonoxid und einer hydroxylierten Co-Reaktante in Anwesenheit eines Katalysatorsystems in Kontakt gebracht wird, welches Katalysatorsystem auf:
a) einer Quelle von Kationen eines oder mehrerer Metalle der Gruppe VIII des Periodensystems;
b) einem Phosphin der allgemeinen Formel PR¹R²R³ oder R²R³M-R-PR¹R², worin R¹ für eine Heteroarylgruppe der Formel 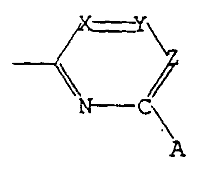 steht, worin A eine funktionelle Gruppe darstellt, die gegenüber Wasserstoff elektronenabziehend ist und worin jeder der Reste X, Y und Z unabhängig voneinander ein Stickstoffatom oder eine Gruppe C-Q bedeutet, wobei Q ein Wasserstoffatom oder einen Substituenten darstellt, R² und R³ unabhängig voneinander eine substituierte oder unsubstituierte (Hetero)hydrocarbylgruppe darstellen oder die zuvor angegebene Bedeutung von R¹ aufweisen, M ein Element der Gruppe VIa, vorzugsweise ein Stickstoff- oder Phosphoratom bedeutet und R eine (substituierte) Hydrocarbyl-Brückengruppe mit 1 bis 4 Kohlenstoffatomen in der Brücke darstellt; und
c) einer Protonensäure aufgebaut ist.

2. Verfahren nach Anspruch 1, worin das Katalysatorsystem auf einem Phosphin beruht, worin R² und R³ unabhängig voneinander eine substituierte oder unsubstituierte Pyridyl-, Alkyl- oder Arylgruppe bedeuten, vorzugsweise eine Phenylgruppe.

3. Verfahren nach Anspruch 1 oder 2, worin das Katalysatorsystem auf einem Phosphin beruht, worin A einen Substituenten darstellt, ausgewählt aus der Gruppe, die aus Halogenatomen und Halogenalkylgruppen mit ein bis drei Kohlenstoffatomen besteht.

4. Verfahren nach Anspruch 3, worin A ein Halogenatom, vorzugsweise ein Chloratom bedeutet.

5. Verfahren nach einem der Ansprüche 1 bis 4, worin das Katalysatorsystem auf einem Phosphin beruht, worin R¹ eine 6-A-2-Pyridylgruppe darstellt.

6. Verfahren nach einem der Ansprüche 1 bis 5, worin das Katalysatorsystem auf einem Monophosphin beruht, vorzugsweise auf Biphenyl (6-chlor-2-pyridyl)phosphin.

7. Verfahren nach einem der Ansprüche 1 bis 6, das in Abwesenheit eines tertiären Amins ausgeführt wird.

8. Verfahren nach einem der Ansprüche 1 bis 7, worin die Molmenge an 1,2-Alkadienverbindung im Einsatzmaterial je Mol acetylenisch ungesättigter Verbindung im Bereich von 0,004 bis 0,05 liegt.

9. Verfahren nach einem der Ansprüche 1 bis 8, worin Methylmethacrylat durch Umsetzen eines Propin und 1,2-Propadien umfassenden Einsatzmaterials mit Kohlenmonoxid und Methanol hergestellt wird.

## Revendications

1. Procédé de carbonylation de composés acétyléniquement insaturés, conformément auquel on met une charge de départ, qui comprend un composé acétyléniquement insaturé et une quantité d'un composé du type 1,2-alcadiène en un rapport molaire de 0,004 à 0,1, en contact et dans des conditions de carbonylation, avec du monoxyde de carbone et un coréactif hydroxylé, en présence d'un système catalytique à base :
a) d'une source de cations d'un ou plusieurs métaux du groupe VIII du tableau périodique,
b) d'une phosphine de la formule générale PR¹R²R³ ou R²R³M-R-PR¹R², dans laquelle R¹ représente un radical hétéroaryle de la formule 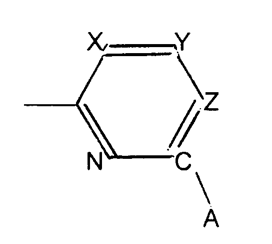 dans laquelle A représente un radical fonctionnel qui est soustracteur d'électrons par rapport à l'hydrogène et chacun des symboles X, Y et Z représente indépendamment un atome d'azote ou un groupe C-Q dans lequel Q représente un atome d'hydrogène ou un substituant, R² et R³ représentent chacun indépendamment un radical (hétéro)hydrocarbyle substitué ou non substitué, ou possèdent les significations attribuées à R¹, M est un élément du groupe Vla, de préférence, un atome d'azote ou un atome de phosphore, R représente un groupe hydrocarbyle (substitué) de pontage possédant de 1 à 4 atomes de carbone dans le pont et
c) d'un acide protonique.

2. Procédé suivant la revendication 1, caractérisé en ce que le système catalytique est basé sur une phosphine dans laquelle R² et R³ représentent chacun indépendamment un groupe aryle, alkyle, ou pyridyle, substitué ou non substitué, de préférence, un groupe phényle.

3. Procédé suivant la revendication 1 ou 2, caractérisé en ce que le système catalytique est basé sur une phosphine dans laquelle A représente un substituant choisi dans le groupe formé par des atomes d'halogènes et des radicaux haloalkyle possédant de un à trois atomes de carbone.

4. Procédé suivant la revendication 3, caractérisé en ce que A représente un atome d'halogène, de préférence, un atome de chlore.

5. Procédé suivant l'une quelconque des revendications 1 à 4, caractérisé en ce que le système catalytique est basé sur une phosphine dans laquelle R¹ représente un groupe 6-A-2-pyridyle.

6. Procédé suivant l'une quelconque des revendications 1 à 5, caractérisé en ce que le système catalytique est basé sur une monophosphine, de préférence. sur la biphényl(6-chloro-2-pyridyl)phosphine.

7. Procédé suivant l'une quelconque des revendications 1 à 6, caractérisé en ce qu'on l'entreprend en l'absence d'une amine tertiaire.

8. Procédé suivant l'une quelconque des revendications 1 à 7, caractérisé en ce que la quantité molaire de composé du type 1,2-alcadiène dans la charge de départ, par mole de composé acétyléniquement insaturé, varie de 0,004 à 0,05.

9. Procédé suivant l'une quelconque des revendications 1 à 8, caractérisé en ce que l'on prépare du méthacrylate de méthyle par la réaction d'une charge de départ comprenant du propyne et du 1,2-propadiène, avec du monoxyde de carbone et du méthanol.
